# EUROPÄISCHE PATENTANMELDUNG

(11) **EP 4 465 068 A1**
(43) Veröffentlichungstag der Anmeldung: **20.11.2024**
(21) Anmeldenummer: 23173593.7
(22) Anmeldetag: 16.05.2023
(51) Int. Cl.: G01R 33/28

(54) **MAGNETRESONANZTOMOGRAPH ZUR LOKALISIERUNG METALLISCHER OBJEKTE UND VERFAHREN ZUM BETRIEB**

(71) Anmelder: Siemens Healthineers AG, 91301 Forchheim (DE)
(72) Erfinder: Faust, Jonas, 71229 Leonberg (DE); Speier, Peter, 91056 Erlangen (DE); Maier, Florian, 91054 Buckenhof (DE)
(74) Vertreter: Siemens Healthineers Patent Attorneys

(57) **Zusammenfassung**

Die Erfindung betrifft einen Magnetresonanztomographen zur Lokalisierung metallischer Objekte und Verfahren zum Betrieb. In einem Schritt des Verfahrens werden Kernspins in einer Umgebung des kompakten metallischen Objektes mittels eines Anregungspulses angeregt. Magnetresonanzdaten werden mit Abtastungen entlang einer Mehrzahl an Trajektorien erfasst, wobei die Abtastungen mittels einer bSSFP-Sequenz erfolgt und die Kernspins mittels eines Gradienten dephasiert werden.

Eine Position eines geometrischen Schwerpunktes des kompakten metallischen Objekts wird anhand einer Position des visuellen Schwerpunktes von erfassten Artefakten ermittelt.

## Beschreibung

Unabhängig vom grammatikalischen Geschlecht eines bestimmten Begriffes sind Personen mit männlicher, weiblicher oder anderer Geschlechteridentität mit umfasst.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden.

Auf vorteilhafte Weise erlaubt ein Magnetresonanztomograph eine Visualisierung des Körperinneren über längere Zeit, ohne den Patienten oder Operateur einer erhöhten Dosis ionisierender Strahlung auszusetzen. Die Magnetresonanztomographie ist wegen der komplexen Bilderfassung und damit langsamen Bildfolge nur schwierig zur Echtzeitüberwachung zu nutzen. Darüber hinaus sind Instrumente in Echtzeit darzustellen, die selbst nicht nur kein Magnetresonanzsignal erzeugen, sondern viel mehr durch ihre metallischen Eigenschaften die Erfassung in der Umgebung des Instruments verhindern.

Es ist daher eine Aufgabe der vorliegenden Erfindung, einen Magnetresonanztomographen und ein Verfahren zum Betrieb des Magnetresonanztomographen bereitzustellen, das eine schnelle Verfolgung eines Instruments erlaubt.

Die Aufgabe wird durch ein erfindungsgemäßes Verfahren gemäß Anspruch 1 und einen erfindungsgemäßen Magnetresonanztomographen nach Anspruch 6 gelöst.

Das erfindungsgemäße Verfahren dient zur Lokalisierung eines kompakten metallischen Objektes mittels eines Magnetresonanztomographen.

Als kompakt in drei Dimensionen wird dabei im Sinne der Erfindung ein Objekt angesehen, dessen Abmessungen sich in allen Raumrichtungen nicht wesentlich, beispielsweise um weniger als 50%, 20 % oder 10% unterscheiden. Ein Idealfall eines derartigen kompakten Objekts ist eine rotationssymmetrische Kugel, wie nachfolgend zu einem Unteranspruch angegeben. Denkbar sind aber beispielsweise auch Ellipsoide oder Polyeder oder auch unregelmäßige Körper, die diese Bedingung erfüllen.

Als lineare, in zwei Dimensionen kompakten metallischen Objekte werden im Sinne der Erfindung Objekte angesehen, die sich entlang einer Achse im Wesentlichen linear erstrecken und deren Abmessungen in einer Ebene senkrecht zu der Achse sich in alle Richtungen in dieser Ebene nicht wesentlich, beispielsweise um weniger als 50%, 20 % oder 10% unterscheiden. Der Idealfall für ein derartiges Objekt ist ein zu der Achse rotationssymmetrischer Zylinder bzw. Hohlzylinder. Denkbar sind aber beispielsweise auch regelmäßige oder unregelmäßige Prismen.

Derartige Objekte sind im Rahmen von Interventionen bzw. der Behandlung eines Patienten häufig eingesetzt. Beispielsweise weist eine Biopsienadel üblicherweise die Form eines Kreiszylinders mit einer Symmetrieachse für eine Rotation um diese Achse, wobei sich die Symmetrieachse mittig entlang der Biopsienadel erstreckt. Auch werden sogenannte Seeds zur Markierung oder Strahlenbehandlung verwendet. Im Falle der Strahlenbehandlung sind die Seeds mit einem strahlenden Isotop gefüllt. Diese Seeds weisen üblicherweise Kugelform oder Zylinderform auf.

In einem Schritt des erfindungsgemäßen Verfahrens werden Kernspins in einer Umgebung des kompakten metallischen Objekts von dem Magnetresonanztomographen durch einen Hochfrequenzpuls zur Präzession um einen Vektor eines Magnetfeldes in der Umgebung des metallischen Objektes angeregt. Das Magnetfeld wird üblicherweise durch das statische Magnetfeld eines Feldmagneten bestimmt, dem zur Schichtselektion ein Gradientenfeld überlagert sein kann. Die Frequenz des Hochfrequenzpulses ergibt sich aus dem magnetischen Moment der Kernspins und der Stärke des Magnetfeldes und wird als Larmorfrequenz bezeichnet. Die Feldstärke und Dauer des Anregungspulses werden durch den zu erzielenden Anregungsgrad bzw. Flipwinkel der Spins bestimmt.

Zur Unterdrückung des Hintergrunds, beispielsweise des umgebenden Gewebes, werden von dem Magnetresonanztomographen in einem Schritt Kernspins durch einen Magnetfeldgradienten dephasiert, die sich nicht in unmittelbarer Umgebung des metallischen Objekts befinden und deshalb nicht durch eine Suszeptibilitätsveränderung durch das kompakte metallische Objekt beeinflusst werden. Ein derartiger Gradient wird auch als White-Marker-Gradient bezeichnet, da er dazu führt, dass nur die unmittelbare Umgebung wegen der von der Suszeptibilitätsschwankung veränderten Larmorfrequenz von dem White-Marker-Gradient nicht dephasiert wird, oder genauer, wirkt der externe White-Marker-Gradient dem lokalen, Suszeptibilitätsinduzierten Gradienten entgegen und dreht die Dephasierung zurueck. So liefert ein Magnetresonanzsignal in unmittelbarer Umgebung des metallischen Objekts ein Signal mit hohem Kontrast gegenüber dem dephasierten Hintergrund.

In einem weiteren Schritt werden von dem Magnetresonanztomographen Magnetresonanzdaten entlang einer Mehrzahl an Trajektorien erfasst. Eine Trajektorie bezeichnet dabei eine Kurve oder auch insbesondere Gerade im k-Raum, entlang derer Magnetresonanzdaten erfasst bzw. abgetastet werden. Vorzugsweise erfolgt das Abtasten in regelmäßig beabstandeten Punkten der Kurve. Im Rahmen der Erfindung wird dabei nicht die Summe aller für die Abbildung erforderlichen Bahnkurven als Trajektorie bezeichnet, sondern einzelne zusammenhängende Abschnitte, die auch als "Readout" oder "Auslese" bezeichnet werden. Für die mit den Unteransprüchen diskutierte radiale Abtastung werden als Trajektorie insbesondere die einzelnen sogenannten Speichen bezeichnet.

Das Abtasten entlang der Trajektorien erfolgt dabei im Rahmen einer balanced steady-state free precession (SSFP)- Sequenz, wie sie beispielsweise aus https://en.wikipedia.org/wiki/Steady-state_free_precession_imaging bekannt ist.

Die Signale der Mehrzahl der Abtastungen werden dabei für eine Abbildung eines durch das kompakte metallische Objekt hervorgerufenen Artefaktes im Ortsraum herangezogen. Durch ein Rekonstruktionsverfahren kann der Magnetresonanztomograph oder ein separater Rekonstruktionsrechner aus den Messwerten im k-Raum eine Abbildung der vom kompakten metallischen Objekt erzeugten Artefakte im Ortsraum erzeugen.

In der Abbildung sind aufgrund der Dephasierung mit dem White-Marker-Puls lediglich die durch das metallische Objekt verursachten Artefakte abgebildet.

Eine Position des kompakten metallischen Objektes wird von dem Magnetresonanztomographen bzw. dessen Steuerung in einem Schritt ermittelt, indem in der Abbildung der visuelle Schwerpunkt der Bildpunkte des Artefaktes bestimmt wird. Bei der visuellen Schwerpunktbildung können die einzelnen Bildpunkte bzw. Voxel mit einer Gewichtungsfunktion multipliziert werden, die vorzugsweise durch den Helligkeitswert des einzelnen Bildpunktes gegeben ist oder in Abhängigkeit davon gebildet wird. Denkbar ist beispielsweise eine Schwellwertfunktion, die den Wert Null annimmt, wenn die Helligkeit eines Bildpunktes unter einem vorbestimmten Schwellwert liegt und den Wert 1, wenn die Helligkeit darüber liegt. Der visuelle Schwerpunkt unterscheidet sich daher von einem Masseschwerpunkt und einem geometrischen Schwerpunkt. Bei Ermitteln eines Masseschwerpunkts wird der Ortsvektor mit einem Masseelement bei der Mittelwertbildung gewichtet. Der geometrische Schwerpunkt ist hingegen von einer Außenkontur bzw. einer Kontur einer Hülle bestimmt und nicht von einer internen Masseverteilung. Für die bevorzugten kompakten metallischen Objekte wie Kugel, Hohlkugel, Nadel und Hohlnadel sind der geometrische Schwerpunkt und der Masseschwerpunkt jedoch identisch.

Die ermittelte Position des visuellen Schwerpunkts des metallischen Objektes stimmt dabei aus den nachfolgend zu den Vorteilen erläuterten Gründen mit der Position des geometrischen Schwerpunkts des Objektes überein.

Auf vorteilhafte Weise eliminiert die Dephasierung mit dem White-Marker-Gradient den Hintergrund durch das umgebende Gewebe, das die Ermittlung der Instrumentenposition durch den visuellen Schwerpunkt des Signals verfälschen würde. Das Artefakt durch das metallische Objekt ist so in der gewonnenen Abbildung von Effekten durch die Umgebung separiert, die so bei nachfolgenden Schritten nicht berücksichtigt werden müssen.

Ohne diesen Hintergrund ist wiederum das Artefakt für sich bei einer Abtastung mittels einer bSSFP-Sequenz entlang der gleichen Trajektorie in entgegengesetzten Richtungen bzw.

über die über den Raumwinkel gleichverteilten Trajektorien jeweils symmetrisch bzw. um den gleichen Weg zu dem wahren Ort verschoben, sodass sich der wahre Ort durch die Mittelung bzw. visuelle Schwerpunktbildung in der Abbildung ergibt.

Dabei hat es sich herausgestellt, dass die bSSFP-Sequenz auf vorteilhafte Weise unabhängig von einer Polarität des White-Marker Gradienten ein symmetrisches White-Marker Artefakt erzeugt. Bei der Verwendung von FLASH Kontrast wäre das Artefakt abhängig von der Polarität des White-Marker Gradienten und eine zweifache Abtastung mit entgegengesetzter Polarität wäre notwendig, um die gleiche Symmetrie wie mit bSSFP herbeizuführen.

Erst durch diese Hintergrundunterdrückung kann durch die einfache Schwerpunktermittlung ohne die detaillierte Kenntnis der Dislokation durch den Suszeptibilitätssprung am metallischen Objekt dessen realer Ort präzise bestimmt werden.

Das Verfahren erlaubt so auf vorteilhafte Weise ohne genaue Kenntnis der durch den Suszeptibilitätssprung hervorgerufenen Dislokation eine Bestimmung der wirklichen Position des abgebildeten Objektes.

Die erfindungsgemäße Vorrichtung teilt die Vorteile des erfindungsgemäßen Verfahrens.

Weitere vorteilhafte Ausführungsformen sind zu den Unteransprüchen angegeben.

In einer denkbaren Ausführungsform des erfindungsgemäßen Verfahrens ist das kompakte metallische Objekt kugelsymmetrisch und die Abtastung eine 3D-Abtastung eines Volumens mit dem Objekt. Mit anderen Worten, die Trajektorien der Abtastung spannen einen dreidimensionalen Raum auf. Beispielsweise ist eine Abtastung entlang von drei Trajektorien denkbar, die zueinander senkrecht sind und ein kartesisches Koordinatensystem angeben.

Auf vorteilhafte Weise kann für ein kugelsymmetrisches oder zumindest annähernd kugelsymmetrisches kompaktes metallisches Objekt, dessen Abmessungen in allen Raumrichtungen im Wesentlichen gleich sind, mit einer einzelnen 3D-Abtastung die Position bestimmt werden. Als kugelsymmetrisch kann ein Objekt beispielsweise angesehen werden, wenn es die zuvor angegebenen Bedingungen für ein in drei Dimensionen kompaktes metallisches Objekt erfüllt, insbesondere gilt es für Kugeln im geometrischen Sinne. Dies ist insbesondere für sogenannte Seeds erfüllt.

In einer möglichen Ausführungsform des erfindungsgemäßen Verfahrens ist das kompakte metallische Objekt ein lineares, in zwei Dimensionen kompaktes metallisches Objekt gemäß der zuvor gemachten Definition. Das kompakte metallische Objekt weist entsprechend eine Achse auf, entlang derer es sich erstreckt. Vorzugsweise weist das Objekt entlang dieser Achse die größte Abmessung auf. Entlang dieser Achse sind zwei Ebenen in einem Abstand zueinander angeordnet. Die Ebenen können parallel zueinander sein, es ist aber auch denkbar, dass diese einen Winkel größer Null Grad miteinander einschließen und sich schneiden. Vorzugsweise hat dann eine Schnittgerade der Ebenen einen Abstand zu der Achse, der größer ist als die Längserstreckung des Objektes oder dem Abstand der Ebenen an der Achse, vorzugsweise größer als ein Mehrfaches der Längserstreckung. Die Steuerung bzw. der Magnetresonanztomograph kann diese Ebenen beispielsweise von einem Nutzer durch eine Eingabe vorgegeben bekommen oder beispielsweise auch durch eine Bilderfassung, gegebenenfalls unter fehlerbehafteter Positionsbestimmung des Objekts festlegen. Erforderlich ist lediglich, dass die Ebenen das Objekt schneiden.

Für jede der Ebenen führt der Magnetresonanztomograph bzw. dessen Steuerung eine 2D-Abtastung mit der bereits beschriebenen bSSFP-Sequenz aus und ermittelt einen Schwerpunkt des abgebildeten Artefakts für jede Ebene. Dadurch erhält der Magnetresonanztomograph zwei geometrische Punkte, durch die sich das lineare kompakte metallische Objekt erstreckt. Wie bereits beschrieben, sind dies reale geometrische Orte, in der eine Ortsverschiebung durch den Suzeptibilitätssprung bereits kompensiert ist. Der Magnetresonanztomograph ermittelt aus diesen beiden Punkten die Achse bzw. eine Gerade durch die Punkte, auf der das lineare kompakte metallische Objekt liegt. bzw. entlang derer es sich erstreckt.

Auf vorteilhafte Weise kann mittels der zwei zweidimensionalen Bilderfassungen mit dem Verfahren eine Ausrichtung des Objekts im Raum präzise bestimmt werden.

In einer denkbaren Ausführungsform des erfindungsgemäßen Verfahrens werden die Magnetresonanzdaten für eine Mehrzahl an Abtastungen entlang radialer Trajektorien erfasst. Dabei sind die Trajektorien im Winkel gleichverteilt über einen Vollkreis in einer Ebene durch das Zentrum des k-Raums bzw. über eine Kugel um das Zentrum des k-Raums. Als radiale Trajektorie wird eine Trajektorie bezeichnet, deren Abtastpunkte entlang einer Geraden durch das Zentrum bzw. den Nullpunkt des k-Raums angeordnet sind. Als gleichverteilt werden Trajektorien bezeichnet, die jeweils einen gleichen Winkelabstand zueinander haben. Beispielsweise sind in 2D bei einem Vollkreis Trajektorien bzw. Geraden gleichverteilt, die einen Winkelabstand von 360 Grad geteilt durch eine natürlichen Zahl n mit n > 2 haben. Die Zahl kann dabei insbesondere auch ungerade sein. Im dreidimensionalen Raum sind beispielsweise Geraden gleichverteilt, die durch das Zentrum und einen Eckpunkt eines gleichmäßigen Polyeders um das Zentrum des Polyeders und des k-Raums verlaufen. Denkbar ist beispielsweise auch eine Spiralanordnung der Ausleserichtung über eine Hemisphäre und dann Umdrehen jeder zweiten Ausleserichtung.

Auf vorteilhafte Weise sind auch die Artefakte eines Objekts in einer Abbildung, die aus Magnetresonanzdaten rekonstruiert wird, die entlang der gleichverteilten Trajektorien erfasst wurden, symmetrisch zu dem Objekt verzerrt bzw. verschoben, sodass sich bei der Schwerpunktbildung diese Verzerrungen aufheben. Dabei führt die unterschiedliche Orientierung der Trajektorien zu einer verbesserten Auflösung im Vergleich zur nachfolgend erläuterten Ausführungsform.

In einer möglichen Ausführungsform des erfindungsgemäßen Verfahrens werden die Magnetresonanzdaten entlang der Trajektorien in beiden Richtungen abgetastet.

Auf vorteilhafte Weise werden durch die Abtastung in beiden Richtungen jeweils wieder auf vorteilhafte Weise symmetrische Verzerrungen und Verschiebungen der Artefakte erzeugt, die sich bei der Schwerpunktbildung aufheben. Allerdings werden bei der Abtastung in zwei Richtungen keine zusätzlichen Informationen erfasst, die zu einer höheren Auflösung führen. Bei vergleichbarer Auflösung im Vergleich zur vorgehend erläuterten Ausführungsform mit einer Gleichverteilung ist daher eine größere Zahl an Abtastungen erforderlich. Dafür sind aber auch Trajektorien denkbar, die nicht durch das Zentrum verlaufen, also beispielsweise auch eine Abtastung in einem Gitter entlang kartesischer Koordinaten.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung einer Ausführungsform eines erfindungsgemäßen Magnetresonanztomographen zur Ausführung des erfindungsgemäßen Verfahrens;
- Fig. 2: einen schematischen Ablaufplan einer Ausführungsform des erfindungsgemäßen Verfahrens;
- Fig. 3: ein schematisches beispielhaftes 3D-Abtastschema gemäß der Erfindung im k-Raum;
- Fig. 4: einen schematischen Ablaufplan einer Ausführungsform des erfindungsgemäßen Verfahrens;
- Fig. 5: ein schematisches beispielhaftes 2D-Abtastschema gemäß der Erfindung;

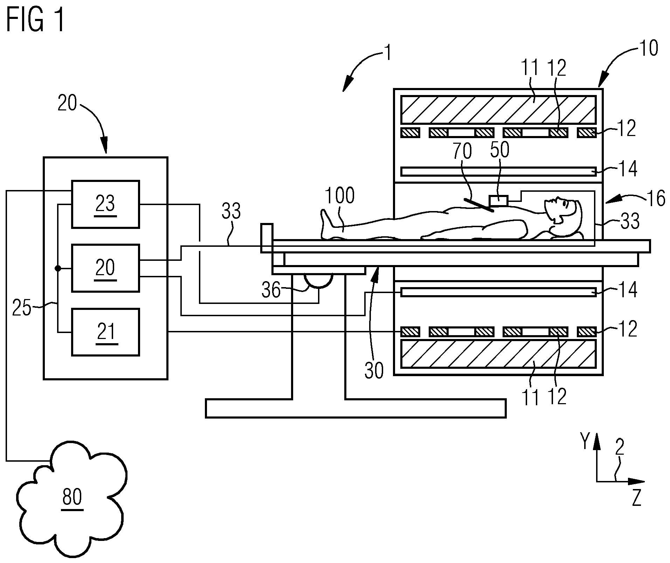

Fig. 1 zeigt eine schematische Darstellung einer Ausführungsform eines erfindungsgemäßen Magnetresonanztomographen 1 zur Ausführung des erfindungsgemäßen Verfahrens.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt.

Eine Patientenliege 30 ist in dem Patiententunnel 16 von der Verfahreinheit 36 bewegbar.

Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu eingerichtet sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu eingerichtet ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben. Im Folgenden bezeichnet der Begriff Sendeantenne eine Antenne, über die das Hochfrequenzsignal zur Anregung der Kernspins ausgestrahlt wird. Dies kann die Körperspule 14 sein, aber auch eine Lokalspule 50 mit Sendefunktion.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu eingerichtet ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die eingerichtet ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Auf dem Patienten 100 ist als eine Lokalspule 50 angeordnet, die über eine Anschlussleitung 33 mit der Hochfrequenzeinheit 22 und deren Empfänger verbunden ist. Denkbar ist es aber auch, dass die Körperspule 14 eine Empfangsantenne im Sinne der Erfindung ist.

Ein kompaktes metallisches Objekt 70, beispielsweise eine Biopsienadel, kann in den Patienten 100 eingeführt werden. Es kann sich aber auch um Objekte 70 im Körper des Patienten 100 handeln, die sich bereits dort befinden und deren Lage erfasst werden soll. Ein Beispiel dafür sind sogenannte Seeds, also Kapseln, die eine Strahlungsquelle enthalten und zur Strahlentherapie genutzt werden. Denkbar sind derartige Seeds auch als Referenzmarke für eine Röntgenuntersuchung oder eine mittels Röntgenaufnahme gesteuerte Bestrahlung. Die genaue geometrische Lage relativ zu einem Tumor ist dabei entscheidend. Das Objekt 70 ist metallisch und erzeugt bei der Magnetresonanzabbildung Artefakte, da zum einen elektromagnetische Wechselfelder abgeschwächt werden. Zum anderen verändert das Metall aufgrund seiner im Vergleich zur Umgebung abweichenden Suszeptibilität auch eine Veränderung der statischen bzw. quasistatischen Magnetfelder, d.h. des Magnetfeldes B0 und der Gradientenfelder. Dies führt dazu, dass an mehreren unterschiedlichen geometrischen Orten in der Umgebung des Objektes gleiche Magnetfeldbeträge auftreten, die über die Ortskodierung auf einen gemeinsamen Punkt abgebildet werden. gleichzeitig wird die Umgebung des Objektes bei einer konventionellen Bilderfassung durch die Variation des Magnetfeldes verzerrt und in eine veränderten Lage zu den umgebenden Organen transponiert.

Der Magnetresonanztomograph 1 kann auch über eine Signalverbindung mit einer externen Signalverarbeitungsressource wie einer Cloud 80 als Teil des Systems verbunden sein, in der Teile des erfindungsgemäßen Verfahrens ausgeführt werden.

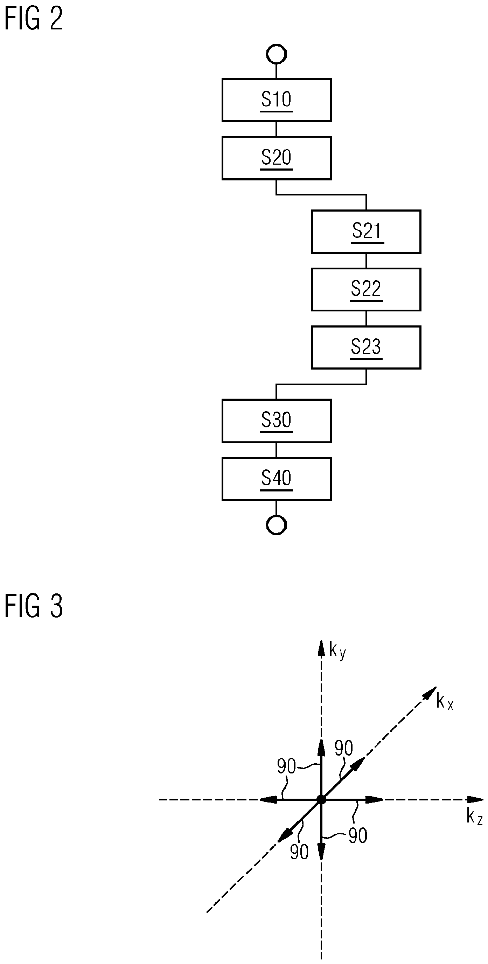

Fig. 2 zeigt einen schematischen Ablaufplan einer Ausführungsform des erfindungsgemäßen Verfahrens.
In einem Schritt S10 regt der Magnetresonanztomograph Kernspins zumindest in einer Umgebung des kompakten metallischen Objektes mittels eines Anregungspulses an. Die Anregung erfolgt erfindungsgemäß entsprechend der verwendeten bSSFP-Sequenz.

Die bSSFP Sequenz zeichnet sich dadurch aus, dass -das nullte Gradientenmoment auf allen Achsen wieder zurückgedreht wird, z.B. dadurch, dass alle Gradienten, die nach dem Anregungspuls ausgespielt werden, vor der nächsten Anregung mit entgegengesetzter Polarität ausgespielt werden.

In einem weiteren Schritt S20 erfasst der Magnetresonanztomograph 1 Magnetresonanzdaten mit einer Abtastung entlang einer radialen Trajektorie 90.

Dabei werden die Kernspins in einem Schritt S21 mittels eines Gradienten dephasiert, um den Hintergrund des Körpers auszublenden. Lediglich die unmittelbare Umgebung des Objekts werden durch die von dem Suszeptibilitätssprung hervorgerufenen Magnetfeldänderungen nicht betroffen, da die Dauer und/oder Stärke des Gradienten gerade so gewählt wird, dass sie die Magnetresonanzsignale im unbeeinflussten, weiter entfernten Gewebe dephasieren.

In einem Schritt S22 werden von dem Magnetresonanztomographen MR-Signale mit einem Abtastschema entlang einer radialen Trajektorie 90 im k-Raum erfasst.

In einem Schritt S23 werden darüber hinaus von dem Magnetresonanztomographen 1 MR-Signale mit einem Abtastschema entlang der radialen Trajektorie 90 in entgegengesetzter Richtung im k-Raum erfasst.

In Fig. 3 sind schematisch radiale Trajektorien 90 im k-Raum dargestellt. Die Trajektorien 90 verlaufen durch das Zentrum bzw. den Koordinatenursprung des k-Raums. In Fig. 3 ist dazu eine 3D-Abtastung dargestellt. Eine Abtastung erfolgt in allen drei Raumkoordinaten des k-Raums und nicht nur in einer Ebene. Beispielhaft sind hier Trajektorien 90 entlang der Koordinatenachsen angegeben. Die Abtastung kann jedoch mit beliebigen radialen Trajektorien 90 erfolgen, die einen dreidimensionalen für die 3D-Abtastung bzw. zweidimensionalen Raum für die 2D-Abtastung in k-Raum aufspannen. Vorzugsweise ist die Zahl der Trajektorien 90 größer, beispielsweise ein dreifaches, zehnfaches oder mehr, und sind über alle Raumrichtungen bzw. Richtungen in der Ebene gleichverteilt.

Auf vorteilhafte Weise führen, wie bereits beschrieben, radiale Trajektorien 90, die über einen Kreis oder eine Vollkugel gleichförmig verteilt sind, zu symmetrischen delokalisierten Artefakten in einem daraus rekonstruierten Bild, die sich bei einer Schwerpunktbildung im Bild gegenseitig aufheben. Dieser Effekt lässt sich aber auch für nicht-radiale, z.B. kartesische Trajektorien erzielen, wenn diese bei der Erfassung der Magnetresonanzsignale in jeweils beide Richtungen entlang der Trajektorie 90 abgetastet werden.

Eine Gleichverteilung der Richtungen der Trajektorie über einen Kreis bzw. eine Kugel erlaubt eine Zeitersparnis bei höherer räumlicher Auflösung gegenüber der Abtastung in entgegengesetzten Richtungen durch Erfassen mehrerer unterschiedlicher k-Raum-Zeilen.

Die Schritte S22 und S23 werden für jede Trajektorie 90 wiederholt.

Anschließend erfolgt in einem Schritt S30 mit den ermittelten MR-Signalen in dem Magnetresonanztomographen 1 oder auf einem externen Gerät in der Cloud 80 eine Bildrekonstruktion, wobei in den erzeugten Bildern das metallische Objekt aufgrund des White-Marker-Gradienten bzw. der Dephasierung das Objekt 70 im Wesentlichen ohne Hintergrund durch das umgebende Gewebe dargestellt ist, allerdings mit den bereits genannten Artefakten wie Verzerrung und Dislokation.
In einem Schritt S40 wird schließlich ein Schwerpunkt der Abbildung des Objektes 70 bestimmt. Vorzugsweise werden dabei die Ortsvektoren mit einem Gewichtungsfaktor beaufschlagt, der beispielsweise einem Helligkeitswert des entsprechenden Pixels oder Voxels entspricht. Denkbar ist auch eine Schwellwertfunktion, die nur Bildpunkten mit einer Amplitude größer einem vorbestimmten Schwellwert berücksichtigt.

Durch radiale Trajektorien 90, die gleichförmig über Kreis bzw. Kugel im k-Raum verteilt sind, erfolgt auch eine über die Richtungen gleichverteilte Verzerrung bzw. Dislokation im Bildraum, sodass bei der anschließenden Mittelwertbildung beim Bestimmen des Schwerpunktes diese sich gegenseitig aufheben und ohne quantitativer Kenntnis der Verzerrung und Dislokation bei der Bestimmung des Schwerpunktes eliminiert werden.

Ein vergleichbarer Effekt lässt sich auch durch das Abtastender Trajektorie 90 in beiden Richtungen erzielen. Bei der verwendeten bSSFP-Sequenz führt dies auf vorteilhafte Weise zu einer symmetrisch gespiegelten Verzerrung bzw. Dislokation im Bildraum, sodass bei der anschließenden Mittelwertbildung beim Bestimmen des Schwerpunktes diese sich gegenseitig aufheben und ohne quantitativer Kenntnis der Verzerrung und Dislokation bei der Bestimmung des Schwerpunktes eliminiert werden.

Das Objekt 70 kann anschließend von dem Magnetresonanztomographen mit der ermittelten wirklichen Position an einen Nutzer ausgegeben werden, beispielsweise in einer Abbildung, oder auch als Navigationshilfe in optischer, akustischer oder taktiler Form.

Der in Fig. 2 dargestellte Ablauf entspricht im Wesentlichen dem erfindungsgemäßen Verfahren für ein kompaktes Objekt 70, dass im Wesentlich in allen Raumrichtungen gleiche Abmessungen und vorzugsweise auch Symmetrien hat, beispielsweise einer Kugel, einem Polyeder oder auch unregelmäßig geformten kompakten Körpern.

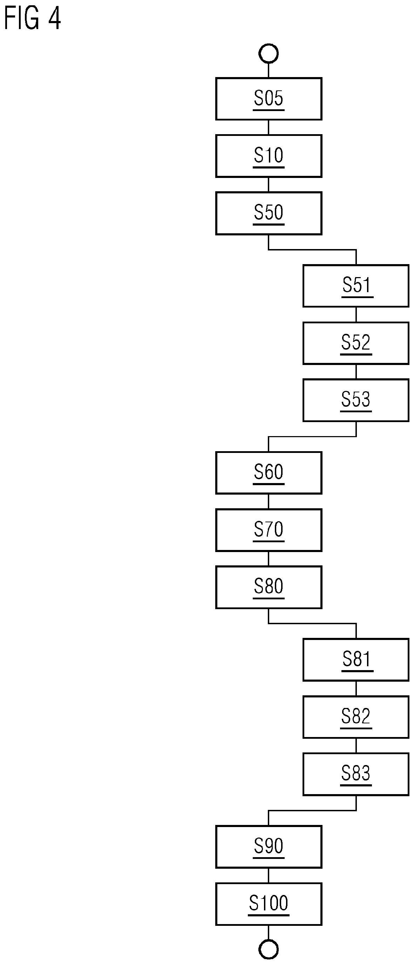

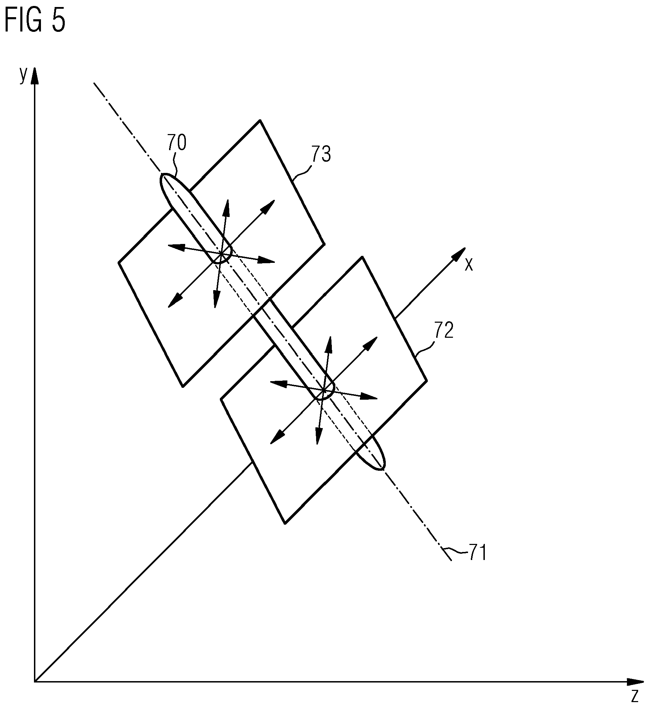

In Fig. 4 ist hingegen ein Ablaufplan einer Ausführungsform des erfindungsgemäßen Verfahrens für ein metallisches Objekt 70 angegeben, wie es in Fig. 5 dargestellt ist. Das Objekt 70 in Fig. 5 erstreckt sich entlang einer Achse 71, wobei die Erstreckung entlang der Achse wesentlich größer ist, als die Abmessungen des Objektes 70 quer zur Achse 71. Im Rahmen der Erfindung wird ein solches Objekt 70 als linear und kompakt in zwei Dimensionen bezeichnet. Dies wird insbesondere aus dem Verfahrensablauf in Fig. 4 ersichtlich, der in weiten Teilen dem für das kompakte Objekt 70 in drei Dimensionen entspricht.

Ein derartiges lineares Objekt 70 lässt sich in seiner Orientierung genau bestimmen, wenn die Achse 71, die eine Gerade ist, durch zwei Punkte genau bestimmt ist. Dies lässt sich wiederum erreichen, indem Schnittpunkte des linearen Objekts 70 mit zwei Ebenen, die zueinander beabstandet sind, ermittelt werden.

Dazu muss der Magnetresonanztomograph 1 diese zwei Ebenen in einem Schritt S05 ermitteln. Der Magnetresonanztomograph 1 kann beispielsweise Daten aus einem Behandlungsplan oder aus einer Bedienereingabe gespeichert haben, die eine grobe, fehlerbehaftete Lage und Position des Objekts 70 angeben. Denkbar ist auch, dass der Magnetresonanztomograph mit einer fehlerbehafteten konventionellen Sequenz der Magnetresonanztomograph 1 die Lage grob bestimmt. Anhand dieser Lage kann der Magnetresonanztomograph 1 mit Mitteln der linearen Algebra eine erste Ebene 72 und eine zweite Ebene 73 ermitteln, die sicher jeweils einen Schnittpunkt mit dem Objekt 70 haben, beispielsweise indem Sicherheitsabstände der Ebenen zu den extremalen Koordinaten des Objektes 70 berücksichtigt werden. Die Ebenen können parallel zueinander und senkrecht zur Achse 71, aber sind auch andere Anordnungen denkbar, solange die Ebenen nicht zusammenfallen oder parallel zur Achse 71 ausgerichtet sind.

In einem Schritt S10 regt der Magnetresonanztomograph Kernspins in einer Umgebung des kompakten metallischen Objektes mittels eines Anregungspulses an. Die Anregung erfolgt erfindungsgemäß entsprechend der verwendeten bSSFP-Sequenz. Im Gegensatz zu dem Verfahren aus Fig. 2 erfolgen in dieser Ausführungsform des Verfahrens lediglich 2D-Abtastungen der Ebenen. Der Anregungspuls für die Ebenen 72, 73 ist dabei vorzugsweise ein Schichtanregungspuls unter entsprechenden Gradientenfeldern zur Selektion einer Schicht in der jeweiligen Ebene.

In einem weiteren Schritt S50 erfasst der Magnetresonanztomograph 1 Magnetresonanzdaten mit einer Abtastung entlang einer Trajektorie 90 in der ersten Ebene 72, wie sie in der Fig. 5 dargestellt sind. Die Trajektorien 90 in der ersten Ebene 72 und der zweiten Ebene 73 sind entsprechend im k-Raum ebenfalls in einer Ebene angeordnet.

Wie bereits zu Fig. 2 erläutert, können die Trajektorien 90 radiale Trajektorien 90 sein und dabei gleichförmig über einen Vollkreis um den Nullpunkt in der Ebene 72, 73 verteilt sein, um den vorteilhaften Effekt der Symmetrie der Artefakte in der Abbildung zu erzielen. Alternativ kann dieser vorteilhafte Effekt auch in einer Ebene 72, 73 erzielt werden, indem die Trajektorie 90 jeweils in beiden Richtungen abgetastet wird, auch wenn es sich nicht um eine radiale Trajektorie 90 handelt, sondern beispielsweise parallel zu den kartesischen Koordinaten verläuft.

Dabei werden die Kernspins in einem Schritt S51 mittels eines Gradienten in der ersten Ebene 72 dephasiert, um den Hintergrund des Körpers auszublenden. Lediglich die unmittelbare Umgebung des Objekts werden durch die von dem Suszeptibilitätssprung hervorgerufenen Magnetfeldänderungen nicht betroffen, da die Dauer und/oder Stärke des Gradienten gerade so gewählt wird, dass sie die Magnetresonanzsignale im unbeeinflussten, weiter entfernten Gewebe dephasieren.

In einem Schritt S52 werden von dem Magnetresonanztomographen MR-Signale mit einem Abtastschema in der ersten Ebene 72 entlang einer radialen Trajektorie 90 im k-Raum erfasst.

In einem Schritt S53 werden darüber hinaus von dem Magnetresonanztomographen MR-Signale mit einem Abtastschema entlang der radialen Trajektorie 90 in entgegengesetzter Richtung im k-Raum erfasst.

Die Schritte S52 und S53 werden für jede Trajektorie 90 in der ersten Ebene 72 wiederholt.

Anschließend erfolgt in einem Schritt S60 mit den ermittelten MR-Signalen in dem Magnetresonanztomographen 1 oder auf einem externen Gerät in der Cloud 80 eine Bildrekonstruktion, hier eine 2D-Bildrekonstruktion für die erste Ebene 72. In den erzeugten Bildern ist das metallische Objekt aufgrund des White-Marker-Gradienten bzw. der Dephasierung das Objekt 70 im Wesentlichen ohne Hintergrund durch das umgebende Gewebe dargestellt ist, allerdings mit den bereits genannten Artefakten wie Verzerrung und Dislokation.

In einem Schritt S70 wird schließlich ein Schwerpunkt der Abbildung des Objektes 70 in der ersten Ebene 72 bestimmt. Vorzugsweise werden dabei die Ortsvektoren mit einem Gewichtungsfaktor beaufschlagt, der beispielsweise einem Helligkeitswert des entsprechenden Pixels entspricht. Denkbar ist auch eine Schwellwertfunktion, die nur Bildpunkten mit einer Amplitude größer einem vorbestimmten Schwellwert berücksichtigt.

Durch die Gleichverteilung der radialen Trajektorien 90 oder das Erfassen der Trajektorien 90 in beiden Richtungen erfolgt bei der verwendeten bSSFP-Sequenz auf vorteilhafte Weise eine symmetrisch gespiegelte Verzerrung bzw. Dislokation im Bildraum, sodass bei der anschließenden Mittelwertbildung beim Bestimmen des Schwerpunktes diese sich gegenseitig aufheben und ohne quantitativer Kenntnis der Verzerrung und Dislokation bei der Bestimmung des Schwerpunktes eliminiert werden.

Die Schritte S50 bis S70 werden als Schritte S80. S81. S82. S83, S90 und S100 in gleicher Weise für die zweite Ebene 73 wiederholt. Es ist auch denkbar, dass die Schritte S50 bis S70 und S80 bis S100 parallel in einer Multischicht-Erfassung für die erste Ebene 72 und die zweite Ebene 73 ausgeführt werden.

Mit den Schwerpunkten in beiden Ebenen sind zwei Orte des linearen Objekts 70 bestimmt. Eine Ausrichtung kann damit als Gerade zwischen den Punkten bestimmt und an den Nutzer ausgegeben werden, beispielsweise in einer Darstellung des Patienten oder über ein Interface als optische, akustische oder taktile Navigationshilfe.

## Patentansprüche

1. Verfahren zur Lokalisierung eines in drei Dimensionen kompakten metallischen Objektes oder linearen, in zwei Dimensionen kompakten metallischen Objekts mittels eines Magnetresonanztomographen (1), wobei das Verfahren die Schritte aufweist:
Anregen von Kernspins in einer Umgebung des kompakten metallischen Objektes (70) mittels eines Anregungspulses;
Erfassen von Magnetresonanzdaten mit Abtastungen entlang einer Mehrzahl an Trajektorien (90), wobei die Kernspins mittels eines Gradienten dephasiert werden,
wobei die Abtastungen mittels einer bSSFP-Sequenz erfolgt,
**dadurch gekennzeichnet, dass**
eine Position eines geometrischen Schwerpunktes des kompakten metallischen Objekts (70) anhand einer Position des visuellen Schwerpunktes von erfassten Artefakten ermittelt wird, die von dem kompakten metallischen Objekt (70) in einer aus den Magnetresonanzdaten rekonstruierten Abbildung erzeugt werden.

2. Verfahren nach Anspruch 1, wobei das kompakte metallische Objekt (70) kugelsymmetrisch ist und die Abtastung eine 3D-Abtastung eines Volumens mit dem kompakten metallischen Objekt (70) ist.

3. Verfahren nach Anspruch 1, wobei das kompakte metallische Objekt (70) ein in zwei Dimensionen kompaktes metallisches Objekt (70) ist, wobei mindestens zwei 2D-Abtastungen in zwei entlang einer Achse (71) einer Längserstreckung des kompakten metallischen Objekts (70) beabstandeten Ebenen (72, 73) erfolgen, wobei sich die Ebenen (72, 73) mit dem kompakten metallischen Objekt (70) schneiden.

4. Verfahren nach einem der vorhergehenden Ansprüche, wobei Magnetresonanzdaten für die Mehrzahl an Abtastungen entlang radialer Trajektorien (90) erfasst werden, wobei die Trajektorien (90) im Winkel gleichverteilt über einen Vollkreis in einer Ebene durch das Zentrum des k-Raums bzw. über eine Kugel um das Zentrum des k-Raums sind.

5. Verfahren nach einem der Ansprüche 1 bis 3, wobei die Trajektorien (90) in beiden Richtungen abgetastet werden.

6. Magnetresonanztomograph zur Lokalisierung eines in drei Dimensionen kompakten metallischen Objektes (70) oder linearen, in zwei Dimensionen kompakten metallischen Objektes (70), wobei der Magnetresonanztomograph (1) eingerichtet ist, Kernspins in einer Umgebung des kompakten metallischen Objektes (70) mittels eines Anregungspulses anzuregen; Magnetresonanzdaten mit Abtastungen entlang einer Mehrzahl an Trajektorien (90) zu erfassen, wobei der Magnetresonanztomograph (1) eingerichtet ist, die Kernspins mittels eines Gradienten zu dephasieren,
wobei der Magnetresonanztomograph (1) eingerichtet ist, die Abtastungen mittels einer bSSFP-Sequenz auszuführen,
**dadurch gekennzeichnet, dass**
der Magnetresonanztomograph (1) eingerichtet ist, eine Position eines geometrischen Schwerpunktes des kompakten metallischen Objekts (1) anhand einer Position des visuellen Schwerpunktes eines erfassten Artefaktes zu ermitteln, das von dem kompakten metallischen Objekt (1) in einer aus den Magnetresonanzdaten rekonstruierten Abbildung erzeugt wird.

7. Magnetresonanztomograph nach Anspruch 6, wobei die Abtastungen 3D-Abtastungen eines Volumens mit dem kompakten metallischen Objekt (70) ist.

8. Magnetresonanztomograph nach Anspruch 6, wobei der Magnetresonanztomograph (1) eingerichtet ist, mindestens zwei 2D-Abtastungen in zwei entlang einer Achse (71) einer Längserstreckung des kompakten metallischen Objekts (70) beabstandeten Ebenen (72, 73) auszuführen, wobei sich die Ebenen (72, 73) mit dem kompakten metallischen Objekt (70) schneiden.

9. Magnetresonanztomograph nach einem der Ansprüche 6 bis 8, wobei der Magnetresonanztomograph (1) eingerichtet ist, Magnetresonanzdaten für eine Mehrzahl an Abtastungen entlang radialer Trajektorien (90) zu erfassen, wobei die Trajektorien (90) im Winkel gleichverteilt über einen Vollkreis in einer Ebene durch das Zentrum des k-Raums bzw. gleichverteilt über eine Kugel um das Zentrum des k-Raums sind.

10. Magnetresonanztomograph nach einem der Ansprüche 6 bis 8, wobei der Magnetresonanztomograph (1) eingerichtet ist, die Trajektorien (90) in beiden Richtungen abzutasten.

11. Computerprogrammprodukt mit Befehlen, die bei Ausführung durch ein Magnetresonanztomographen (1) nach einem der Ansprüche 6 bis 10 das Magnetresonanztomographiesystem (1) dazu veranlassen, ein Verfahren nach einem der Ansprüche 1 bis 5 durchzuführen.
